# FASCICULE DE BREVET EUROPEEN

(11) **EP 2 247 745 B1**
(45) Date de publication et mention de la délivrance du brevet: **04.11.2015**
(21) Numéro de dépôt: 09711108.2
(22) Date de dépôt: 12.02.2009
(51) Int. Cl.: C12P 21/06, A23L 1/305, A23J 3/34, A61K 38/01, A61K 35/60

(54) **Hydrolysat de protéines de poissons présentant une activité de stimulation et de maintien du capital osseux, compositions nutraceutiques et pharmacologiques comprenant un tel hydrolysat et procédé d'obtention**
Fischproteinhydrolysat mit knochenstimulierender und knochenerhaltender Wirkung, nutrazeutische und pharmakologische Zusammensetzung mit einem derartigen Hydrolysat sowie Verfahren zu seiner Gewinnung
Fish protein hydrolysate having a bone-stimulating and maintaining activity, nutraceutical and pharmacological compositions comprising such a hydrolysate and method for obtaining same

(30) Priorité: 12.02.2008 FR 0800753
(43) Date de publication de la demande: 10.11.2010
(73) Titulaire: Compagnie Des Peches Saint Malo Sante, 35400 Saint-Malo (FR)
(72) Inventeur: DRIEU LA ROCHELLE, Hubert, 35400 Saint-Malo (FR); COUROIS, Elisa, 35400 Saint-Malo (FR)
(74) Mandataire: Maillet, Alain
(86) Numéro de dépôt international: PCT/EP2009/051655
(87) Numéro de publication internationale: WO 2009/101146

(56) Documents cités:
- EP-A- 1 273 239
- FR-A- 2 835 703
- GB-A- 2 136 002
- US-A1- 2005 164 950
- RAVALLEC-PLE ROZENN ET AL: "The presence of bioactive peptides in hydrolysates prepared from processing waste of sardine (Sardina pilchardus)" JOURNAL OF THE SCIENCE OF FOOD AND AGRICULTURE, vol. 81, no. 11, 1 septembre 2001 (2001-09-01), pages 1120-1125, XP002501882 ISSN: 0022-5142
- GUERARD F ET AL: "Production of tuna waste hydrolysates by a commercial neutral protease preparation" JOURNAL OF MOLECULAR CATALYSIS. B, ENZYMATIC, ELSEVIER, AMSTERDAM, NL, no. 19-20, 2 décembre 2002 (2002-12-02), pages 489-498, XP002293270 ISSN: 1381-1177
- ASPMO S I ET AL: "Enzymatic hydrolysis of Atlantic cod (Gadus morhua L.) viscera" PROCESS BIOCHEMISTRY, ELSEVIER, NL, vol. 40, no. 5, 1 avril 2005 (2005-04-01), pages 1957-1966, XP004755275 ISSN: 1359-5113
- WU HUI-CHUN ET AL: "Free amino acids and peptides as related to antioxidant properties in protein hydrolysates of mackerel (Scomber austriasicus)." FOOD RESEARCH INTERNATIONAL, vol. 36, no. 9-10, 2003, pages 949-957, XP002502818 ISSN: 0963-9969
- REBECA B D ET AL: "PRODUCTION OF FISH PROTEIN HYDROLYSATES WITH BACTERIAL PROTEASES YIELD AND NUTRITIONAL VALUE" JOURNAL OF FOOD SCIENCE, vol. 56, no. 2, 1991, pages 309-314, XP002502820 ISSN: 0022-1147
- KRISTINSSON HORDUR G ET AL: "Biochemical and functional properties of atlantic salmon (Salmo salar) muscle proteins hydrolyzed with various alkaline proteases" JOURNAL OF AGRICULTURAL AND FOOD CHEMISTRY, vol. 48, no. 3, mars 2000 (2000-03), pages 657-666, XP002502822 ISSN: 0021-8561
- DATABASE WPI Week 200261 Thomson Scientific, London, GB; AN 2002-569182 XP002534786 & JP 2002 142723 A (NIPPON TENNENBUTSU KENKYUSHO KK) 21 mai 2002 (2002-05-21)

## Description

La présente invention concerne un hydrolysat de protéines de poisson présentant une activité biologique d'intérêt, en particulier un effet sur la stimulation et le maintien du capital osseux. L'invention concerne encore un procédé d'obtention d'un tel hydrolysat de protéines de poisson, une composition alimentaire, un complément alimentaire ainsi qu'un médicament comprenant un tel hydrolysat de protéines de poisson.

Le tissu osseux est un tissu conjonctif spécialisé qui est composé notamment de cellules ostéoblastes, ostéocytes et ostéoclastes, de fibres de collagène et d'une matrice minéralisée. Les ostéoblastes sont responsables de la synthèse du tissu osseux, en particulier de la synthèse de la matrice osseuse constituée de collagène de type I, de protéoglycanes et de glycoprotéines, alors que les ostéoclastes sont responsables de la résorption osseuse, c'est-à-dire la dégradation du tissu osseux.

Le tissu osseux est en constant renouvellement grâce à un processus dynamique de remodelage intervenant tout au long de la vie. Ce remodelage est assuré grâce à l'action conjuguée des ostéoclastes et ostéoblastes via les Basic Multicellular Unit (BMU). Ces unités ont une durée de vie d'environ 6 mois et permettent de renouveler environ 10% du squelette par an.

Un cycle de remodelage débute par l'activation d'un nouveau BMU sur une surface inactive de l'os. Les cellules bordantes de la lignée osseuse vont alors disparaître et être remplacées par des ostéoclastes qui creusent une lacune sur la surface endostéale de l'os pendant environ 2 semaines. C'est la phase de résorption. Une fois terminée, les ostéoclastes sont détruits par apoptose et les pré-ostéoblastes se différencient. Les ostéoblastes matures peuvent alors synthétiser la nouvelle matrice osseuse, notamment en élaborant en permanence l'osséine, composant organique de l'os. Puis cette matrice est peu à peu minéralisée et des ostéoblastes se retrouvent piégés au coeur de la matrice, devenant ainsi des ostéocytes. (Hadjidakis et al., 2006).

L'ostéoporose, ou la baisse du capital osseux, est un phénomène observé dans l'ensemble d'une population vieillissante. « L'ostéoporose est une affection généralisée du squelette caractérisée par une diminution de la densité osseuse et une altération de la microarchitecture du tissu osseux responsable d'une augmentation de la fragilité de l'os et, par conséquent, du risque de fracture » (définition de l'OMS, 1992). Il s'agit d'une maladie touchant préférentiellement les femmes mais du fait de l'allongement de leur espérance de vie, les hommes ne sont plus épargnés. Cette maladie est connue pour entraîner, par exemple, des tassements vertébraux, des fractures du poignet ou des fractures du col du fémur. Les conséquences des fractures du col du fémur sont importantes puisqu'elles s'accompagnent de 20% de mortalité dans l'année qui suit la fracture et dans 50% des cas de séquelles graves et invalidantes.

A la ménopause, la déficience en oestrogènes provoque une baisse de l'ostéoformation, une détérioration de l'architecture de l'os et une perte de la masse osseuse entraînant ainsi un risque de fracture important à long terme. Du fait de cette déficience en oestrogènes, le renouvellement osseux s'accélère (augmentation du nombre de BMU), provoquant l'apparition d'une porosité plus importante. La résorption osseuse augmente fortement par rapport à la formation, qui elle, n'est pas modifiée. Ceci est principalement du à une augmentation de la durée de vie des ostéoclastes.

Dans le cadre démographique actuel d'un pays tel que la France, où l'espérance de vie ne cesse de progresser, l'ostéoporose est aujourd'hui un problème majeur de santé publique.

L'importance du rôle joué par l'alimentation dans l'acquisition du capital osseux et son maintien semble aujourd'hui ouvrir la voie d'une véritable prévention de cette pathologie. Ainsi, une première approche est la modification du style de vie comprenant des changements diététiques. La prise de suppléments diététiques seule ou associée à des modifications du style de vie est bénéfique pour conduire à un maintien du capital osseux.

Le document US2005/164950 divulgue l'utilisation de peptides pour réduire ou inhiber les symptomes de l'ostéoporose. Le document EP1273239 décrit l'utilisation d'un hydrolysat partiel de collagène de poisson pour le traitement de l'ostéoporose.

Les demandeurs ont alors découvert que des hydrolysats de protéines de poissons, ou peptidiques, obtenus à partir de l'hydrolyse enzymatique d'une source de protéines composée de certains poissons possédaient des propriétés de stimulation de la croissance de la masse osseuse. Plus particulièrement, les demandeurs ont découvert que de tels hydrolysats étaient capables d'activer la croissance des cellules ostéoblastes et d'inhiber la croissance des cellules ostéoclastes.

L'invention concerne ainsi un hydrolysat de protéines de poisson qui se caractérise en en ce qu'il est obtenu par hydrolyse enzymatique d'au moins une source de protéines choisie parmi les espèces de poissons pélagiques *Micromesistius poutassou, Clupea harengus*, *Scomber scombrus*, *Sardina pilchardus, Trisopterus esmarki*, *Trachurus spp.,* de poissons démersaux *Gadus morhua*, *Pollachius virens*, *Melanogrammus aeglefinus, Coryphaenoides rupestris,* de poissons appartenant à l'ordre des siluriformes, ladite hydrolyse enzymatique étant réalisée par une enzyme endopeptidase dérivée de *Bacillus subtilis* et en ce qu'il présente les caractéristiques physico-chimiques suivantes :
- la répartition du profil moléculaire suivant : de 33 à 39 % de molécules de poids moléculaire inférieur à 300 Da, de 34 à 37 % de molécules dont le poids moléculaire est compris entre 300 et 1 000 Da, de 21 à 24 % de molécules dont le poids moléculaire est compris entre 1 000 et 3 000 Da, de 3 à 4 % de molécules dont le poids moléculaire est compris entre 3 000 et 5 000 Da et de 1 à 2 % de molécules dont le poids moléculaire est compris entre 5 000 et 10 000 Da,
- la teneur en lipides inférieure à 1 %, en pourcentage de produit brut,
- la teneur en glucides inférieure à 4 %, en pourcentage de produit brut,
- la teneur en protéines est supérieure à 80 %, en pourcentage de produit brut,
- la teneur en matière minérale est comprise entre 5 et 10 %, de produit brut.

L'hydrolysat de protéines selon l'invention permet le maintien de la masse osseuse ou la stimulation de la croissance osseuse. L'hydrolysat de protéines selon l'invention peut être utilisé dans la prévention ainsi que dans le traitement de maladies telles que l'ostéoporose, par exemple post ménopausique, la déminéralisation osseuse, la malabsorption calcique, la malabsorption de la vitamine D, les maladies du métabolisme osseux. L'hydrolysat de protéines selon l'invention permet une stimulation de la croissance des cellules ostéoblastes et une inhibition de la croissance des cellules ostéoclastes comme le démontreront les exemples qui suivent.

Selon une caractéristique de l'invention, ledit hydrolysat de protéines de poisson présente la composition en acides aminés suivante : Acide glutamique 16,9 %, Acide aspartique 11,7 %, Lysine 10 %, Leucine 8,2 %, Arginine 6,3 %, Alanine 6,8 %, Valine 4,8 %, Isoleucine 4,4 %, Glycine 5 %, Thréonine 4,5 %, Sérine 4,4 %, Tyrosine 3,2 %, Phénylalanine 3,9 %, Méthionine 2,6 %, Proline 3,4 %, Histidine 2 %, Cystine 1 %, Tryptophane 0,8 %, en pourcentage en poids par rapport au poids total d'acides aminés.

Selon un mode préféré de réalisation de l'invention, ladite source de protéines de poisson comprend la pulpe du filet dudit ou desdits poissons.

La présente invention concerne encore un procédé d'obtention d'un hydrolysat de protéines à partir d'une source de protéines de poisson, ledit hydrolysat possédant des propriétés de maintien ou de stimulation de la croissance de la masse osseuse. Le procédé selon l'invention se caractérise en ce qu'il comprend:
- le broyage d'au moins une source de protéines choisie parmi les espèces de poissons pélagiques *Micromesistius poutassou, Clupea harengus, Scomber scombrus*, *Sardina pilchardus, Trisopterus esmarki, Trachurus spp.,* de poissons démersaux *Gadus morhua, Pollachius virens, Melanogrammus aeglefinus, Coryphaenoides rupestris,* de poissons appartenant à l'ordre des siluriformes, en présence d'eau, de manière à récupérer la pulpe de poisson,
- l'hydrolyse enzymatique de ladite source de protéines à une température comprise entre 50 et 75°C, pendant 1 à 5 heures, après l'ajout d'une enzyme endopeptidase dérivée de *Bacillus subtilis* de manière à obtenir un mélange réactionnel,
- l'arrêt de ladite hydrolyse enzymatique par inactivation de ladite enzyme après élévation de la température dudit mélange réactionnel à un niveau non inférieur à 70°C, pendant 8 à 20 minutes.
- la séparation de l'hydrolysat de protéines obtenu du reste du mélange réactionnel.

L'hydrolyse enzymatique de la pulpe des poissons précités selon le procédé selon l'invention permet l'obtention d'un hydrolysat de protéines de poisson présentant des propriétés de régulation de la croissance de la masse osseuse. L'hydrolyse enzymatique est réalisée par une enzyme soigneusement sélectionnée pour permettre d'obtenir un hydrolysat de protéines possédant les propriétés recherchées et précitées. Le procédé, de par la nature de l'enzyme, la température d'hydrolyse ainsi que l'absence de solvants, respecte les qualités organoleptiques et nutritionnelles de l'hydrolysat obtenu. Cet hydrolysat peut être alors incorporé dans des compositions alimentaires ou des préparations pharmaceutiques.

Selon une caractéristique de l'invention, une enzyme endopetidase dérivée de *Bacillus subtilis* est le produit Corolase N commercialisé par la société AB Enzyme (Feldbergstraße 78, D-64293, Darmstadt, Germany).

De préférence, ledit broyage de la source de protéines est réalisé en présence d'eau selon un rapport source de protéines/eau de 1.

Selon un mode de réalisation de l'invention, ladite hydrolyse enzymatique est réalisée selon un ratio enzyme/source de protéines compris entre 0,1 et 1 %. Préférentiellement, le ratio enzyme/source de protéines est égal à 0,75 %.

Selon un mode de réalisation de l'invention, ladite hydrolyse enzymatique est réalisée à une température de 55°C.

La séparation de l'hydrolysat de protéines obtenu du reste du mélange réactionnel est généralement réalisée par centrifugation à une vitesse comprise entre 4000 et 7000 RPM et élimination du culot obtenu.

Avantageusement, la séparation de l'hydrolysat de protéines obtenu est réalisée par filtration dudit mélange réactionnel préalablement à ladite centrifugation. La filtration du milieu réactionnel permet d'éliminer les matières solides.

Selon un mode de réalisation de l'invention, ledit procédé comprend, en outre, la concentration et l'atomisation ou la lyophilisation dudit hydrolysat obtenu.

Selon un autre mode de réalisation de l'invention, l'arrêt de ladite hydrolyse enzymatique est réalisé par élévation de la température dudit mélange réactionnel jusqu'à 85°C et maintien de cette température pendant 15 minutes.

Selon un mode de réalisation de l'invention, ledit broyage de ladite source de protéines est réalisé à partir du filet dudit ou desdits poissons.

La présente invention concerne encore un hydrolysat de protéines de poisson obtenu par un procédé tel que décrit précédemment. Un tel hydrolysat est tel que défini précédemment.

La présente invention concerne encore une composition, un complément alimentaire ainsi qu'une composition alimentaire comprenant un hydrolysat de protéines de poisson tel que décrit précédemment.

La présente invention concerne encore un médicament comprenant un hydrolysat de protéines de poisson tel que décrit précédemment, ainsi que l'utilisation d'un tel hydrolysat de protéines de poisson pour la fabrication d'un médicament pour le traitement ou la prévention de l'ostéoporose, par exemple post ménopausique, la déminéralisation osseuse, la malabsorption calcique, la malabsorption de la vitamine D, les maladies du métabolisme osseux.

L' hydrolysat de protéines de poissons selon l'invention est encore utilisé pour dans la stimulation de la croissance d'ostéoblastes et l'inhibition de la croissance d'ostéoclastes.

Les formulations nutraceutiques ou pharmaceutiques incorporant un hydrolysat de protéines de poisson selon l'invention, pourront comprendre des ingrédients couramment utilisés dans ce type de formulations tels que des liants, des agents de saveurs, des conservateurs, des colorants, et pourront, dans le cas de compléments alimentaires ou de médicaments, se présenter sous la forme de comprimés, granulés ou gélules. Des formulations selon l'invention pourront également se présenter sous la forme de produits alimentaires tels que des boissons, ou encore sous la forme de suspensions ou de sirops.

Les caractéristiques de l'invention mentionnées ci-dessus, ainsi que d'autres, apparaîtront plus clairement à la lecture de la description suivante d'un exemple de réalisation, ledit exemple se voulant illustratif et non limitatif.

La Fig. 1 illustre la répartition des poids moléculaires des fragments protéiques d'un hydrolysat de protéine de merlan bleu selon l'invention, et

Les Figs. 2 à 5 illustrent les répartitions des poids moléculaires des fragments protéiques d'hydrolysats de protéine d'autres espèces de poisson selon l'invention,

Les Figs. 6 et 7 illustrent l'effet sur la croissance de cellules osseuses d'un hydrolysat de protéine de merlan bleu selon l'invention,

La Fig. 8 illustre l'inhibition de la croissance des ostéoclastes par un hydrolysat de protéine de merlan bleu selon l'invention,

La Fig. 9 illustre un effet d'un hydrolysat de protéine de merlan bleu selon l'invention sur la densité minérale osseuse chez la souris.

### Exemple 1: Hydrolysat de protéines obtenu à partir de merlan bleu

Le merlan bleu (*Micromesistius poutassou*) est pêché en Atlantique Nord au large de Terre-Neuve. Les poissons sont débités en filets qui sont ensuite broyés de manière à en obtenir la pulpe. Cette pulpe de poisson constitue une source de protéines pour la production de l'hydrolysat. La pulpe est conservée à -20°C jusqu'à utilisation.

Trois kilos de la pulpe de merlan bleu préalablement décongelée sont mélangés à de l'eau selon un rapport massique de 1. La température est portée à 55°C et une enzyme endopeptidase dérivée de *Bacillus subtilis*, commercialisée sous le nom de Corolase N par la société AB Enzyme (Feldbergstraße 78, D-64293, Darmstadt, Germany) est alors ajoutée dans le mélange réactionnel selon un ratio enzyme/source de protéines de 0,75 %.

La réaction d'hydrolyse est menée pendant 2 heures puis l'enzyme est inactivée par élévation de la température du milieu réactionnel à 85°C. Cette température est maintenue durant 15 minutes.

L'hydrolysat de protéines de merlan bleu obtenu, dénommé par la suite H1, est ensuite filtré sur un tamis (grillage de 2mm/2mm) de manière à éliminer les matières solides, puis récupéré dans un récipient. La fraction récupérée dans le récipient est alors centrifugée pendant 30 minutes +- 5 minutes, à une vitesse comprise entre 4000 et 7000 RPM. Après élimination du culot, le surnageant est récupéré, lyophilisé et conservé dans un endroit frais et sec, à l'abri de la lumière. Le surnageant peut également être atomisé.

### Analyses physico-chimiques de l'hydrolysat de proteines H1 obtenu à partir de merlan bleu

Une détermination des poids moléculaires des peptides constitutifs de l'hydrolysat de protéines H1 est réalisée par chromatographie d'exclusion stérique (SEC-HPLC).

L'hydrolysat de protéines sous forme de poudre après lyophilisation est suspendu dans de l'eau ultra pure à raison de 20 mg/mL, puis filtré sur une membrane de 0,45µm et analysé par filtration sur gel avec une colonne Superdex Peptide HR 10/30, commercialisée par la société Pharmacia. La matrice de la colonne est composée d'un gel poreux réticulé (diamètre 13-15µm) d'agarose et de dextrane d'un volume total de 24 mL. Son domaine de fractionnement est compris entre 100 et 7000 Da. La colonne est montée sur une chaîne HPLC, commercialisée par la société Dionex, qui est équipée d'une pompe (module Dionex P680). La mesure est réalisée par un détecteur d'ultra violets multi longueur d'onde (module Dionex UVD 170 U). L'hydrolysat de protéines HMB est élué par une phase mobile contenant de l'acétonitrile, de l'eau et du TFA. L'élution dure environ 1H à un débit de 0.5mL/min.

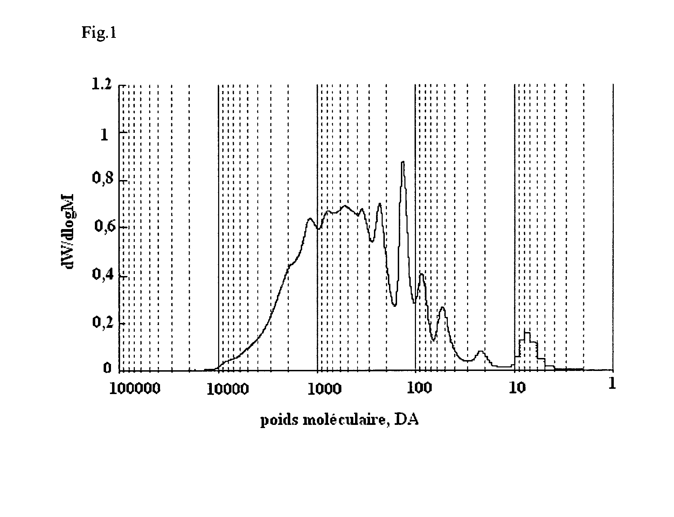

La répartition des poids moléculaires est calculée à partir des paramètres d'une droite de calibration obtenue après le passage dans la colonne de marqueurs de poids moléculaires connus suivants: Cytochrome C (12 400 Da), aprotinine (6 511 Da), gastrine I (2 126 Da), substance P fragment 1-7 (1 348 Da), glycine (75 Da) et leupeptine (463 Da). Les données sont collectées grâce au logiciel Chromeleon (Dionex). Les pourcentages des poids moléculaires sont calculés à l'aide d'un logiciel (GPC Cirrus de chez Polymer Laboratories). La longueur d'onde d'acquisition est de 214 nm. La répartition des poids moléculaires en fonction dW/logM est donnée par le logiciel et est représentée sur la Fig.1. Le pourcentage de l'aire sous la courbe correspond au pourcentage de molécules. La répartition des poids moléculaires par classe de taille est donnée dans le Tableau 1:

**Tableau 1**

| Classes | H1 | H2 | H3 | H4 | H5 | H6 | H7 | H8 | H10 | H9 | H11 |
|---|---|---|---|---|---|---|---|---|---|---|---|
| < 0,3 | 33-39 | 38 | 33 | 33 | 33 | 35 | 33 | 33 | 33 | 36 | 34 |
| 0,3-1 | 34-37 | 37 | 37 | 37 | 37 | 37 | 37 | 37 | 36 | 37 | 37 |
| 1-3 | 21-24 | 21 | 24 | 24 | 24 | 22 | 24 | 24 | 24 | 22 | 23 |
| 3-5 | 3-4 | 3 | 4 | 4 | 4 | 4 | 4 | 4 | 4 | 3 | 4 |
| 5->10 | 1-2 | 1 | 2 | 2 | 2 | 2 | 2 | 2 | 2 | 2 | 2 |

La composition en acides aminés de l'hydrolysat de protéines H1 est donnée dans le Tableau 2 et est obtenu en suivant les indications de la Directive Européenne 98/64/CE et de la norme NF EN ISO 13904-octobre 2005.

**Tableau 2**

| **Acide aminé** | **Pourcentage d'acide aminé** | **Acide aminé** | **Pourcentage d'acide aminé** |
|---|---|---|---|
| Acide glutamique | 16,9 | Glycine | 5 |
| Lysine | 10 | Thréonine | 4,5 |
| Acide aspartique | 11,7 | Sérine | 4,4 |
| Leucine | 8,2 | Tyrosine | 3,2 |
| Arginine | 6,3 | Phénylalanine | 3,9 |
| Alanine | 6,8 | Méthionine | 2,6 |
| Valine | 4,8 | Proline | 3,4 |
| Isoleucine | 4,4 | Histidine | 2 |
| Cystine | 1 | Tryptophane | 0,8 |

La teneur en protéines est supérieure à 80 %, en pourcentage de produit brut (norme NF V18-120-Mars 1997 corrigée KJELDAHL).

La teneur en lipides est inférieure à 1%, en pourcentage de produit brut (selon directive européenne 98/64/CE).

La valeur énergétique de l'hydrolysat de protéines H1 est d'environ 350 Kcal/100g.

La teneur en glucides est inférieure à 4 % (déduite à partir des teneurs en protéines et glucides et de la valeur énergétique).

### Exemple 2: Hydrolysat de protéines obtenu à partir d'autres espèces de poisson selon l'invention

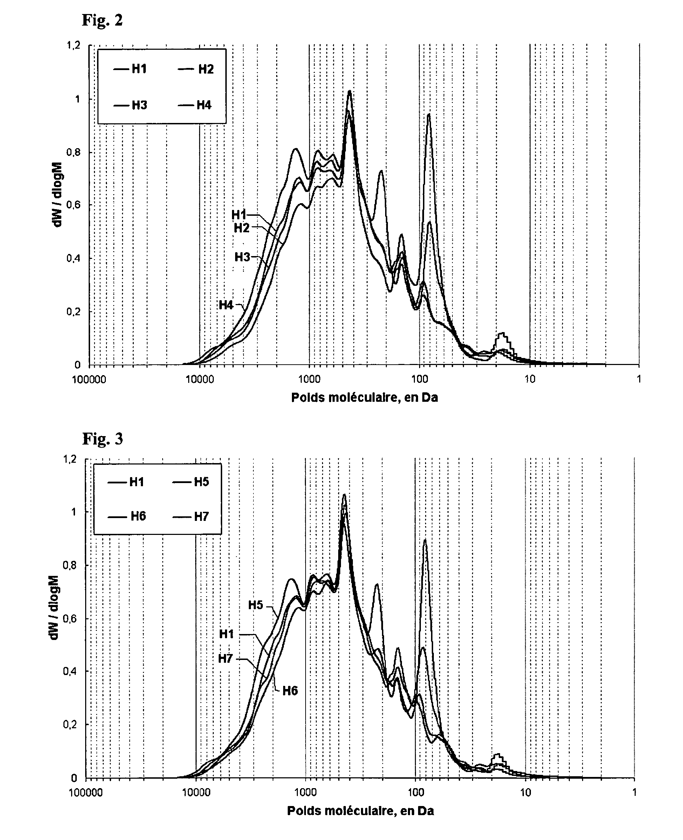

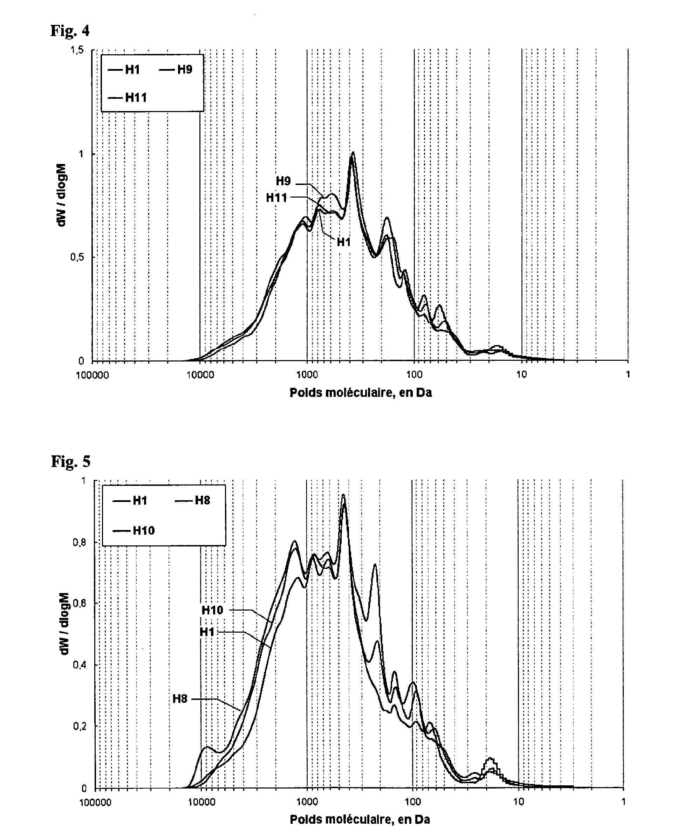

Des hydrolysats de protéines de maquereau (H2) (scomber scombrus), chinchard (H3) (Trachurus spp.), grenadier (H4) (*Coryphaenoides rupestris*) (Fig. 2); tacaud (H5) (*Trisopterus esmarki*), sardine (H6) (*Sardina pilchardus*), hareng (H7) (*Clupea harengs*) (Fig. 3). panga (H8) et (Suliforme) lieu noir (H10) (*Pollachius virens*) (Fig. 5); cabillaud (H9) (*Gadus morhua*) et églefin (H11) (*Melanogrammus aeglefinus*) (Fig. 4) ont été préparés selon le procédé de l'exemple 1. La répartition des poids moléculaires des peptides composant chaque hydrolysat a été analysée selon le même procédé que celui utilisé dans l'exemple 1.

La répartition des poids moléculaires en fonction dW/logM est donnée sur les Figs. 2 à 5, et la répartition des poids moléculaires par classe de taille est donnée dans le Tableau 1, *supra*. Le pourcentage de l'aire sous la courbe correspond au pourcentage de molécules peptidiques.

Tous les hydrolysats montrent un profil de répartition des poids moléculaires identique.

### Exemple 4: Activités biologigues de l'hydrolysat de protéines H1 Etude in vitro:

L'hydrolysat de protéines H1 a été testé vis-à-vis de sa capacité à favoriser la stimulation de la croissance des cellules ostéoblastes et à provoquer une inhibition de la croissance des cellules ostéoclastes sur des cultures cellulaires *in vitro*.

L'hydrolysat de protéines H1 sous forme de poudre après lyophilisation a été suspendu dans de l'eau ultra pure à raison de 20 mg/mL, puis filtré sur une membrane de 0,22 micron pour le stériliser. Différentes dilutions ont été alors réalisées: 1/10 (soit la concentration la plus élevée en peptides, c'est-à-dire, 2mg/mL), 1/20 (1mg/mL), 1/50 (0,4 mg/mL), 1/100 (0,2 mg/mL), 1/500 (0,04 mg/mL) et 1/1000 (0,02 mg/mL).

### Cultures cellulaires:

Des cultures de cellules osseuses mixtes, c'est-à-dire constituées d'ostéoblastes et d'ostéoclastes, issues de souris de la souche BalB/c ont été réalisées comme suit.

Les souris ont été tuées par décapitation et les os des pattes arrière ont été prélevés dans des conditions d'asepsie puis placés dans un tube stérile contenant du milieu de culture alpha-Milieu Essentiel Minimum (MEM-α) (1X) liquide, supplémenté en sels de Earle, en glutamax I (Invitrogen Corporation, 1600 Faraday Ave., Carlsbad, CA 92008), en ribonucléosides et en désoxyribonucléosides.

Les os des pattes ont été grattés au scalpel puis réduits en petits morceaux afin d'extraire la moelle osseuse. Les cellules et tous les débris ont été recueillis dans un tube, lequel a été soumis à une agitation vigoureuse pendant environ 2 minutes afin de détacher les cellules des débris osseux. La suspension ensuite a été filtrée sur un filtre d'une porosité de 70µm. Les cellules ont été récupérées par centrifugation (5 minutes à 800g), le surnageant a été éliminé et le culot cellulaire a été récupéré dans un milieu de culture complet (α-MEM supplémenté avec de la pénicilline/streptomicine, 10% de Sérum de Veau Foetal et 10⁻⁸ de 1α,25-(OH)₂D₃ ou 1α,25-dihydroxyvitamine D₃).

Les cellules obtenues ainsi à partir de 4 ou 5 tibias ont été ensemencées dans un flacon de 75 cm². Les flacons de cultures ont été incubés à 37°C sous atmosphère humide contenant 5% de CO2. Le temps nécessaire pour l'adhésion des cellules à la surface du flacon est de 5 heures environ. Après cette période nécessaire pour l'attachement des précurseurs des ostéoblastes et des ostéoclastes, le milieu a été remplacé à raison de 15 mL pour une surface de 75 cm². Le milieu a été changé deux fois par semaine. Après 6 jours de culture, le milieu a été aspiré. Les cellules ont été détachées du flacon à l'aide d'un grattoir puis mises en suspension à une densité de 1,2.10⁴ cellules/mL. Ces conditions de culture permettent la croissance des ostéoblastes et des ostéoclastes.

Le lendemain, le milieu a été changé et l'hydrolysat de protéines H1 a été ajouté au milieu de culture selon les différentes dilutions citées précédemment (1/10, 1/20, 1 /50, 1/100, 1/500 et 1/1000).

Une solution de sérum d'albumine bovin (BSA) (20mg/ml) a été préparée en tant que témoin (témoin BSA) et ajoutée aux cellules dans les mêmes conditions de dilution que les autres solutions protéiques.

Un témoin de culture (témoin négatif) est utilisé et correspond à une culture de cellules osseuses dans laquelle ni l'hydrolysat de protéines H1, ni de la BSA n'ont été ajoutés.

### Croissance cellulaire d'une culture de cellules mixtes : quantification de l'ADN:

La quantification de l'ADN permet d'évaluer l'effet des différentes protéines, H1 ou BSA, sur la croissance cellulaire des cultures de cellules mixtes.

Il s'agit d'un dosage fluorométrique réalisé à l'aide du kit FluoReporter Blue Fluorometric dsDNA Quantitation Kit (F-2962) (Molecular Probes; Invitrogen Corp.).

Les cellules incubées en présence des différentes dilutions de l'hydrolysat de protéines H1, les cellules incubées en présence de la solution témoin de BSA, ainsi que les cellules des cultures témoins, sont lysées par congélation pour libérer l'ADN. Le réactif de Hoechst 33258 utilisé dans le kit est spécifique des régions riches en séquences A-T de l'ADN double brin nouvellement synthétisé, auxquelles il se lie. La liaison est détectée par fluorescence.

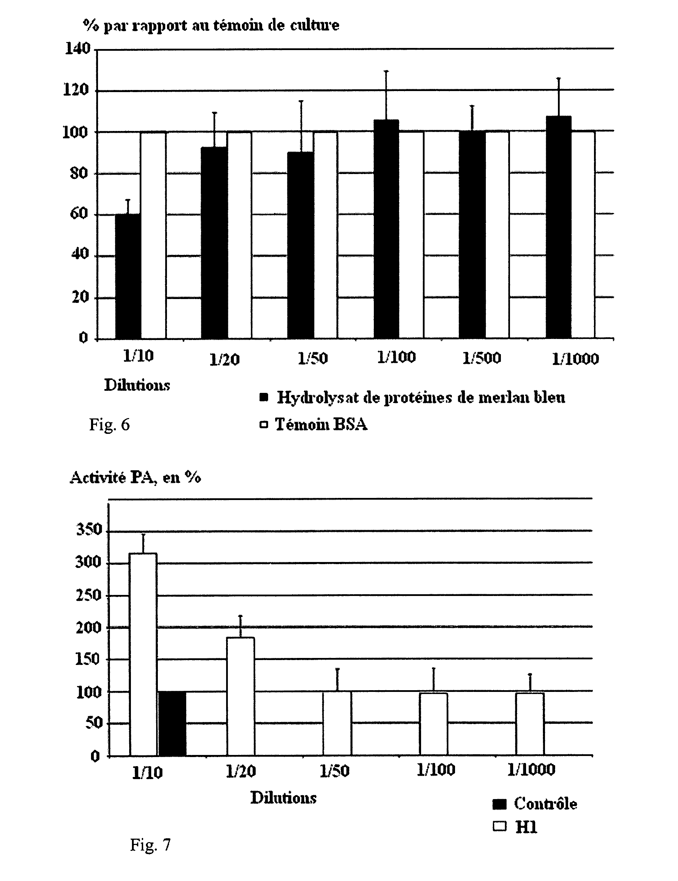

La Fig. 6 présente l'effet de l'hydrolysat de protéines H1 à différentes concentrations sur la croissance des cellules osseuses de souris de la souche BalB/c. Les résultats sont exprimés en pourcentage par rapport au témoin de culture.

L'hydrolysat de protéines H1 inhibe significativement la croissance des cellules osseuses (inhibition de 40 % au 1/10). En effet, en présence d'une solution de protéine de référence, le BSA, la croissance des cellules osseuses est de 100 % alors qu'en présence d'une solution d'hydrolysat de protéines H1 dilué au 1/10, la croissance des cellules osseuses est de 60%.

Afin de déterminer si les cellules, dont la croissance est inhibée, sont des ostéoclastes ou des ostéoblastes, une analyse complémentaire a été réalisée. Cette analyse est basée sur le dosage de la phosphatase alcaline qui est sécrétée spécifiquement par les ostéoblastes. Ainsi, en mesurant l'activité de la phosphatase alcaline et en exprimant les résultats obtenus en fonction de la quantification de l'ADN précédemment décrite, il est possible de déterminer si l'hydrolysat de protéines H1 est favorable au développement des ostéoblastes et inhibe les ostéoclastes.

### Stimulation de la croissance des ostéoblastes : dosage de la phosphatase alcaline

Le 4-méthylumbelliféryle phosphate (4-MUP) (Sigma-Aldrich Corporation, 3050 Spruce St., St. Louis, MO 63103) est un substrat de la phosphatase alcaline.

La déphosphorylation du substrat par la phosphatase alcaline est mesurée par fluorescence.

Le substrat a été ajouté dans les milieux des cultures de cellules dans lesquelles ont été ajoutées au préalable les différentes dilutions de l'hydrolysat de protéines H1 ainsi que dans le milieu de culture de cellules témoin (témoin de culture).

La Fig. 7 présente l'activité de la phosphatase alcaline (PA) exprimé en %, en fonction des différentes dilutions de l'hydrolysat de protéines H1 et également en l'absence de l'hydrolysat de protéines (témoin de culture).

Sachant qu'une valeur de 100 % d'activités de phosphatase alcaline est obtenue pour le témoin de culture, nous constatons que les dilutions au 1/10 et au 1/20 de l'hydrolysat de protéines H1 sont capables de stimuler la différenciation des ostéoblastes. La dilution au 1/10 multiplie par trois l'activité de la phosphatase alcaline et par conséquent augmente la différenciation des ostéoblastes par trois par rapport au témoin de culture. Compte tenu de cela, il apparaît que l'inhibition de la croissance des cellules osseuses observée précédemment concernait les ostéoclastes et non les ostéoblastes.

### Inhibition de la croissance des ostéoclastes : digestion d'un substrat osseux

Les cultures primaires de cellules mixtes de souris ont été cultivées sur un substrat osseux synthétique. Les ostéoclastes matures présents dans les cultures de cellules mixtes sont capables de digérer un substrat osseux.

L'hydrolysat de protéines H1, dilué au 1/10^{ème}, a été ajouté au milieu de culture des cellules.

Un contrôle correspondant à une culture de cellules sur le substrat osseux en l'absence de l'hydrolysat de protéines H1 a été réalisé.

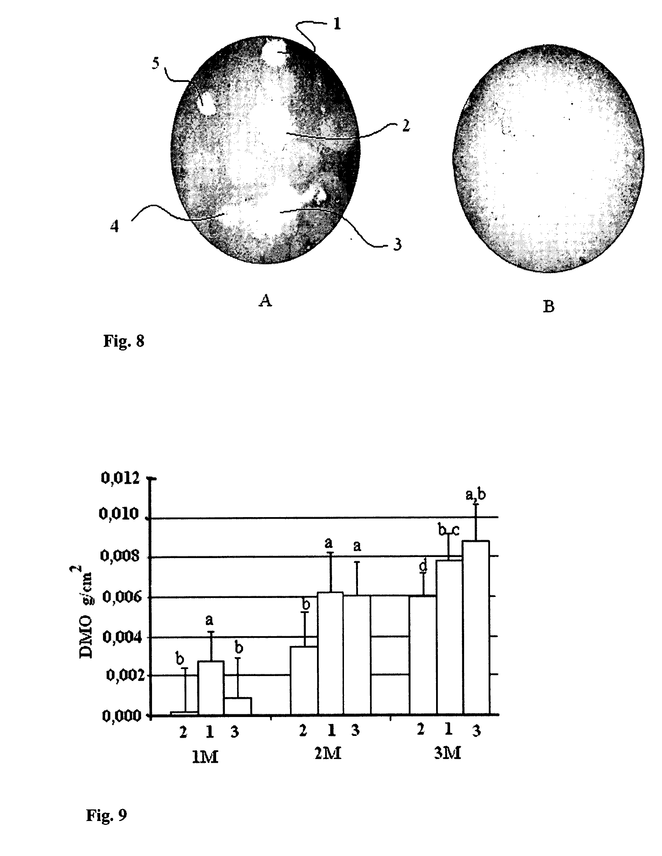

La Fig. 8 présente deux photographies qui ont été réalisées au microscope en présence de l'hydrolysat de protéines H1 (Fig. 8A) ou en l'absence de l'hydrolysat de protéines H1 (contrôle, Fig. 8B).

Les photographies montrent des zones de digestions (1, 2, 3, 4, 5) visibles sur le substrat osseux de contrôle (Fig. 8A) alors que le substrat osseux visible sur la Fig. 8B n'est pas digéré. L'hydrolysat de protéines H1 a donc diminué l'activité des ostéoclastes. Ces résultats, combinés à ceux de la quantification de l'ADN présentés précédemment, permettent de conclure que l'hydrolysat de protéines H1 inhibe la croissance des ostéoclastes.

### Exemple 5: Activités biologigues de l'hydrolysat de protéines H1 Etude in vivo:

Lors de cette étude, un modèle de souris ovariectomisées a été utilisé afin d'induire une déficience en oestrogènes et de mimer un phénomène de ménopause. Un régime contenant 14% en poids d'hydrolysats de protéines de poisson H1 a été comparé à un régime standard témoin contenant 14% en poids de protéines totales de lait.

Les évolutions de la densité minérale osseuse totale ont été suivies au cours du temps.

36 souris C3H/Hen de 10 semaines, fournies par le centre d'élevage Harlan, ont été placées dans des cages au sein d'une pièce thermostatée à 22°C en cycle jour-nuit inversé (nuit de 6h à 18h). Les animaux ont été habitués à leur environnement et à leur nourriture sous forme de poudre pendant 2 semaines. Chaque animal a reçu de l'eau et de la nourriture *ad libitum* (5 grammes par jour et par souris) pendant les 3 mois de l'étude.

A douze semaines, les souris ont subi une ovariectomie ou une chirurgie sans ablation des ovaires. Suite à l'opération, les animaux ont été répartis en 3 groupes de 12 suivant les régimes qu'ils allaient recevoir :
- Groupe 1 (témoin positif). Les souris sont opérées sans subir d'ablation des ovaires et reçoivent le régime contrôle, soit 14% de protéines totales de lait (caséine et lactosérum inclus).
- Groupe 2 (témoin négatif). Les souris subissent l'ovariectomie et reçoivent le régime contrôle.
- Groupe 3. Les souris sont opérées pour subir une ablation des ovaires et reçoivent un régime contenant 14% de H1 (même composition que le régime contrôle mais les protéines de lait sont remplacées par les hydrolysats de protéines de poisson obtenus dans l'exemple 1).

Tous les régimes fournis contiennent 14% de protéines et sont de composition énergétique proche comme l'indique le tableau 4 ci-dessous.

**Tableau 3**

| | **Constituants en g**/**kg de régime groupes 1 et 2** | **Constituants en g/kg de régime groupe** 3 |
|---|---|---|
| **Protéines totales de lait** | 140,0 | 0,0 |
| **H1 (poudre)** | 0,0 | 140,0 |
| **Total Kcal/kg de régime** | **3671,4** | **3582,8** |

### Suivi de l'évolution de la densité minérale osseuse (DMO):

La densité osseuse est le reflet de la qualité de l'os et de sa résistance. Cette mesure, servant de référence pour le diagnostic de l'ostéoporose, est effectuée par absorptiométrie biphotonique utilisant les rayons X (DEXA : Dual Energy X-ray absorptiométrie). L'appareil, le PIXImus "densitometer" ?(LUNAR CORPORATION, Madison, WI), émet des rayons X qui traversent le corps de la souris et des capteurs détectent l'atténuation de l'intensité du faisceau de rayons X à travers les régions osseuses calcifiées. Il détermine également la surface et la masse de tissus osseux calcifiés et de tissus « mous » traversés. Ainsi, la masse maigre, la masse grasse et la DMO totale (quantité d'os en grammes sur la surface d'os du corps en cm²) peuvent être évaluées. Pour effectuer les mesures, les souris sont anesthésiées (par un mélange xylazine/kétamine) et placées dans une coupelle en plastique sur le plateau de l'appareil. Celui-ci réalise une image, qui est retranscrite sur un écran. Les images obtenues peuvent ensuite être retraitées en se focalisant sur une partie précise du corps de la souris : comme au niveau des fémurs et de la colonne vertébrale. En effet, ces os étant riches en os spongieux, ils sont plus facilement affectés par l'ostéoporose.

La DMO a été évaluée juste avant l'ovariectomie, ainsi que tous les mois suivant l'opération. Toutes ces mesures ont été réalisées au sein de l'IFR 02 de l'UFR Médicale Paris 7.

### Résultats

La Fig. 9 montre le gain cumulé de densité minérale osseuse (DMO) au cours du temps et en fonction des groupes (1, 2 et 3). Les valeurs sont données sous forme de moyenne ± écart-type. Les groupes avec des lettres différentes sont statistiquement différents (p<0,05).

Au temps 0, avant ovariectomie, les groupes avaient une DMO comparable entre eux. Afin d'avoir une meilleure idée de l'effet des régimes, les gains de DMO cumulés ont été étudiés. Dans tous les cas, la DMO augmente au cours du temps. Après un mois de régime (1M), l'effet opération est maximal puisque le groupe 1 a une DMO significativement supérieure à tous les autres groupes c'est-à-dire que ces souris continuent à acquérir de l'os alors que la croissance osseuse est stoppée chez les animaux qui subissent une déficience en oestrogènes. Après deux mois de régime (2M), la DMO des groupes 3 rattrape celle du groupe 1 et est significativement différente de celle du groupe 2. Donc pour ces deux groupes, un effet régime est bien observé dès deux mois.

Enfin, à 3 mois de régime (3M), les groupes 1 et 2 sont toujours statistiquement différents. Les animaux du groupe 3 ont un gain de DMO significativement plus important que ceux du groupe 2 et ne sont pas statistiquement différents de ceux du groupe 1.

L'ablation des ovaires induit une chute de la densité minérale osseuse. Les différences entre le groupe 2 (ovariectomisé) et le groupe 1 perdurent au cours du temps prouvant l'incidence de la déficience en oestrogènes sur la minéralisation de l'os et la validité du modèle. Enfin, le groupe 3 augmente de façon très significative la minéralisation osseuse, dépassant le gain de DMO des groupes 1 et 2 témoins. Il y a donc bien un effet de la fraction protéique d'hydrolysat de poisson H1 sur la minéralisation osseuse totale. Ces résultats confirment ceux obtenus précédemment lors de l'étude *in vitro* qui démontrait la capacité des hydrolysats selon l'invention à augmenter la différenciation des ostéoblastes tout en diminuant celle des ostéoclastes (exemple 4).

## Revendications

1. Hydrolysat de protéines de poisson **caractérisé en ce qu'**il est obtenu par hydrolase enzymatique d'au moins une source de protéines choisie parmi le groupe composé des poissons *Micromesistius poutassou, Clupea harengus, Scomber scombrus, Sardina pilchardus, Trisopterus esmarki, Trachurus spp., Gadus morhua, Pollachius virens, Melanogrammus aeglefinus, Coryphaenoides rupestris,* des poissons appartenant à l'ordre des siluriformes, ladite hydrolyse enzymatique étant réalisée par une enzyme endopeptidase dérivée de *Bacillus subtilis* et **en ce qu'**il présente:
- la répartition du profil moléculaires suivant : de 33 à 39 % de molécules de poids moléculaire inférieur à 300 Da, de 34 à 37 % de molécules dont le poids moléculaire est compris entre 300 et 1 000 Da, de 21 à 24 % de molécules dont le poids moléculaire est compris entre 1 000 et 3 000 Da, de 3 à 4 % de molécules dont le poids moléculaire est compris entre 3 000 et 5 000 Da et de 1 à 2 % de molécules dont le poids moléculaire est compris entre 5 000 et 10 000 Da,
- une teneur en lipides inférieure à 1 %, en pourcentage de produit brut,
- une teneur en glucides inférieure à 4 %, en pourcentage de produit brut,
- une teneur en protéines supérieure à 80 %, en pourcentage de produit brut.
- une teneur en matière minérale comprise entre 5 et 10 %, en pourcentage de produit brut.

2. Hydrolysat de protéines de poisson selon la revendication 1, **caractérisé en ce qu'**il est obtenu par hydrolyse enzymatique d'une source de protéines composée de *Micromesistius poutassou* et qu'il présente la composition en acides aminés suivante : Acide glutamique 16,9 %, Acide aspartique 11,7 %, Lysine 10 %. Leucine 8,2 %, Arginine 6,3 %, Alanine 6,8 %, Valine 4,8 %, Isoleucine 4,4 %, Glycine 5 %, Thréonine 4,5 %, Sérine 4,4 %, Tyrosine 3,2 %. Phénylalanine 3,9 %. Méthionine 2,6 %, Proline 3,4 %, Histidine 2 %, Cystine 1 %, Tryptophane 0.8 %, en pourcentage en poids par rapport au poids total d'acides aminés.

3. Procédé d'obtention d'un hydrolysat de protéines de poissons tel que défini dans les revendications 1 à 2, **caractérisé en ce qu'**il comprend:
- le broyage d'au moins une source de protéines choisie parmi le groupe composé des espèces de poissons *Micromesistius poutassou, Clupea harengus. Scomher scombrus, Sardina pilchardus, Trisopterus esmarki*, *Trachurus spp*., *Gadus morhua, Pollachius virens, Melanogrammus aeglefinus, Coryphaenoides rupestris,* de poissons appartenant à l'ordre des siluriformes, en présence d'eau, de manière à récupérer la pulpe dudit ou desdits poissons,
- l'hydrolyse enzymatique de ladite source de protéines à une température comprise entre 50 et 75°C, pendant 1 à 5 heures, après l'ajout d'une enzyme endopeptidase dérivée de *Bacillus subtilis* de manière à obtenir un mélange réactionnel,
- l'arrêt de ladite hydrolyse enzymatique par inactivation desdites enzymes après élévation de la température dudit mélange réactionnel à un niveau non inférieur à 70°C, pendant 8 à 20 minutes.
- la séparation de l'hydrolysat de protéines obtenu du reste du mélange réactionnel.

4. Procédé selon l'une des revendications 3, **caractérisé en ce que** ladite hydrolyse enzymatique est réalisée selon un ratio enzyme source de protéines compris entre 0.01 et 2%, préférentiellement égal à 0,75 %, et à une température d'hydrolyse égale à 55°C.

5. Procédé selon l'une des revendications 3 à 4, **caractérisé en ce que** ledit broyage de ladite source de protéines est réalisé à partir du filet dudit, ou desdits poissons.

6. Produit alimentaire **caractérisé en ce qu'**il comprend un hydrolysat de protéines de poissons tel que défini dans l'une des revendications 1 à 2.

7. Complément alimentaire en ce qu'il comprend un hydrolysat de protéines de poissons tel que défini dans l'une des revendications 1 à 2.

8. Composition pharmaceutique **caractérisée en ce qu'**elle comprend un hydrolysat de protéines de poissons tel que défini dans les revendications l'une des revendications 1 à 2.

9. Composition **caractérisée en ce qu'**elle comprend un hydrolysat de protéines de poissons tel que défini dans les revendications l'une des revendications 1 à 2.

10. Médicament **caractérisé en ce qu'**il comprend un hydrolysat de protéines de poissons tel que défini dans les revendications l'une des revendications 1 à 2.

11. Utilisation d'un hydrolysat de protéines de poisson tel que défini dans l'une des revendications 1 à 2 pour la fabrication d'un médicament destiné au traitement ou la prévention d'une maladie choisie parmi l'ostéoporose, la déminéralisation osseuse, la malabsorption calcique, la malabsorption de la vitamine D, les maladies du métabolisme osseux.

12. Utilisation selon la revendication 11, dans laquelle l'ostéoporose est post ménopausique.

13. Hydrolysat de protéines de poissons selon l'une des revendications 1 à 2 pour son utilisation dans le traitement ou la prévention d'une maladie choisie parmi l'ostéoporose, la déminéralisation osseuse, la malabsorption, calcique, la malabsorption de la vitamine D. les maladies du métabolisme osseux.

14. Hydrolysat de protéines de poissons pour son utilisation selon la revendication 13, **caractérisé en ce que** l'ostéoporose est post ménopausique.

15. Hydrolysat de protéines de poissons pour son utilisation selon la revendication 13, **caractérisé en ce que** le traitement ou la prévention de ladite maladie inclut la stimulation de la croissance d'ostéoblastes et l'inhibition de la croissance d'ostéoclastes.

## Patentansprüche

1. Proteinhydrolysat aus Fisch, **dadurch gekennzeichnet, dass** es durch enzymatische Hydrolyse erhalten wird aus wenigstens einer Quelle von Proteinen ausgewählt aus der Gruppe bestehend aus den Fischen *Micromesistius poutassou, Clupea harengus, Scomber scombrus, Sardina pilchardus, Trisopterus esmarki, Trachurus spp., Gadus morhua, Pollachius virens, Melanogrammus aeglefinus, Coryphaenoides rupestris,* Fischen, die der Ordnung Siluriformes angehören, wobei die enzymatische Hydrolyse mit einem Endopeptidaseenzym ausgeführt wird, das aus *Bacillus subtilis* stammt, und dass es Folgendes aufweist:
- die folgende Molekularprofilverteilung: 33 bis 39 % Moleküle eines Molekulargewichts von weniger als 300 Da, 34 bis 37 % Moleküle, deren Molekulargewicht zwischen 300 und 1.000 Da liegt, 21 bis 24 % Moleküle, deren Molekulargewicht zwischen 1.000 und 3.000 Da liegt , 3 bis 4 % Moleküle, deren Molekulargewicht zwischen 3.000 und 5.000 Da liegt sowie 1 bis 2 % Moleküle, deren Molekulargewicht zwischen 5.000 und 10.000 Da liegt,
- einen Gehalt an Lipiden von weniger als 1 %, als Prozentgehalt des Trockenprodukts,
- einen Gehalt an Kohlenhydraten von weniger als 4 %, als Prozentgehalt des Trockenprodukts,
- einen Gehalt an Proteinen von mehr als 80 %, als Prozentgehalt des Trockenprodukts,
- einen Gehalt an mineralischem Material zwischen 5 und 10 %, als Prozentgehalt des Trockenprodukts.

2. Proteinhydrolysat aus Fisch nach Anspruch 1, **dadurch gekennzeichnet, dass** es durch enzymatische Hydrolyse einer Quelle gemischter Proteine von *Micromesistius poutassou* erhalten wird und dass es die folgende Aminosäurezusammensetzung aufweist:
Glutaminsäure 16,9 %, Asparaginsäure 11,7 %, Lysin 10 %, Leucin 8,2 %, Arginin 6,3 %, Alanin 6,8 %, Valin 4,8 %, Isoleucin 4,4 %, Glycin 5 %, Threonin 4,5 %, Serin 4,4 %, Tyrosin 3,2 %, Phenylalanin 3,9 %, Methionin 2,6 %, Prolin 3,4 %, Histidin 2 %, Cystein 1 %, Tryptophan 0,8 %, als Gewichtsprozent im Verhältnis zum Gesamtgewicht der Aminosäuren.

3. Verfahren zum Erhalt eines Proteinhydrolysats aus Fischen wie in den Ansprüchen 1 bis 2 definiert, **dadurch gekennzeichnet, dass** es Folgendes umfasst:
- das Zerkleinern wenigstens einer Quelle von Proteinen, die ausgewählt ist aus der Gruppe bestehend aus den Fischarten *Micromesistius poutassou, Clupea harengus, Scomber scombrus, Sardina pilchardus, Trisopterus esmarki, Trachurus spp., Gadus morhua, Pollachius virens, Melanogrammus aeglefinus, Coryphaenoides rupestris,* Fischen, die der Ordnung Siluriformes angehören, in Gegenwart von Wasser, um das Mark des Fisches oder der Fische zu gewinnen,
- die enzymatische Hydrolyse der Quelle von Proteinen bei einer Temperatur zwischen 50 und 75 °C während 1 bis 5 Stunden, anschließend das Hinzufügen eines Endopeptidaseenzyms, das aus *Bacillus subtilis* stammt, so dass ein Reaktionsgemisch erhalten wird,
- das Stoppen der enzymatischen Hydrolyse durch Inaktivierung der Enzyme nach Erhöhung der Temperatur des Reaktionsgemischs auf eine Höhe von nicht weniger als 70 °C während 8 bis 20 Stunden,
- das Trennen des erhaltenen Proteinhydrolysats vom Rest des Reaktionsgemischs.

4. Verfahren nach Anspruch 3, **dadurch gekennzeichnet, dass** die enzymatische Hydrolyse nach einem Enzym/Quelle von Proteinen-Verhältnis zwischen 0,01 und 2 % ausgeführt wird, vorzugsweise 0,75 % und bei einer Hydrolysetemperatur von 55 °C.

5. Verfahren nach einem der Ansprüche 3 bis 4, **dadurch gekennzeichnet, dass** das Zerkleinern der Quelle von Proteinen ausgehend von einem Filet des Fischs oder der Fische durchgeführt wird.

6. Nahrungsmittelprodukt, **dadurch gekennzeichnet, dass** es ein Proteinhydrolysat aus Fischen wie in einem der Ansprüche 1 bis 2 definiert umfasst.

7. Nahrungsergänzungsmittel, **dadurch gekennzeichnet, dass** es ein Proteinhydrolysat aus Fischen wie in einem der Ansprüche 1 bis 2 definiert umfasst.

8. Pharmazeutische Zusammensetzung, **dadurch gekennzeichnet, dass** sie ein Proteinhydrolysat aus Fischen wie in einem der Ansprüche 1 bis 2 definiert umfasst.

9. Zusammensetzung, **dadurch gekennzeichnet, dass** sie ein Proteinhydrolysat aus Fischen wie in einem der Ansprüche 1 bis 2 definiert umfasst.

10. Arzneimittel, **dadurch gekennzeichnet, dass** es ein Proteinhydrolysat aus Fischen wie in einem der Ansprüche 1 bis 2 definiert umfasst.

11. Verwendung eines Proteinhydrolysats aus Fisch wie in einem der Ansprüche 1 bis 2 definiert zur Herstellung eines Arzneimittels, das zur Behandlung oder Vorbeugung einer Erkrankung vorgesehen ist, die ausgewählt wird aus Osteoporose, Knochendemineralisierung, Calcium-Malabsorption, Malabsorption von Vitamin D, Erkrankungen des Knochenstoffwechsels.

12. Verwendung nach Anspruch 11, wobei die Osteoporose postmenopausal ist.

13. Proteinhydrolysat aus Fischen nach einem der Ansprüche 1 bis 2 zur Verwendung bei der Behandlung oder die Vorbeugung einer Erkrankung, die ausgewählt wird aus Osteoporose, Knochendemineralisierung, Calcium-Malabsorption, Malabsorption von Vitamin D, Erkrankungen des Knochenstoffwechsels.

14. Proteinhydrolysat aus Fischen zur Verwendung nach Anspruch 13, **dadurch gekennzeichnet, dass** die Osteoporose postmenopausal ist.

15. Proteinhydrolysat aus Fischen zur Verwendung nach Anspruch 13, **dadurch gekennzeichnet, dass** die Behandlung oder die Vorbeugung der Erkrankung die Stimulierung des Osteoblastenwachstums und die Hemmung des Osteoklastenwachstums umfasst.

## Claims

1. Fish protein hydrolysate, **characterised in that** it is obtained by enzymatic hydrolysis of at least one protein source selected from the group comprising the fish *Micromesistius poutassou, Clupea harengus, Scomber scombrus, Sardina pilchardus, Trisopterus esmarki, Trachurus spp., Gadus morhua, Pollachius virens, Melanogrammus aeglefinus, Coryphaenoides rupestris,* and fish belonging to the order siluriformes, said enzymatic hydrolysis being performed by an endopeptidase enzyme derived from *Bacillus subtilis,* and **in that** it has:
- the following molecular profile distribution: 33 to 39% of molecules having a molecular weight of less than 300 Da., 34 to 37% of molecules whose molecular weight is between 300 and 1000 Da, 21 to 24% of molecules whose molecular weight is between 1000 and 3000 Da, 3 to 4% of molecules whose molecular weight is between 3000 and 5000 Da, and 1 to 2% of molecules whose molecular weight is between 5000 and 10,000 Da,
- a lipid content of less than 1%, as a percentage of the crude product,
- a glucide content of less than 4%, as a percentage of the crude product,
- a protein content of more than 80%, as a percentage of the crude product,
- a content of mineral matter of between 5 and 10%, as a percentage of the crude product.

2. Fish protein hydrolysate according to claim 1, **characterised in that** it is obtained by enzymatic hydrolysis of a protein source comprising *Micromesistius poutassou* and **in that** it has the following amino acid composition: glutamic acid 16.9%, aspartic acid 11.7%, lysine 10%, leucine 8.2%, arginine 6.3%, alanine 6.8%, valine 4.8%, isoleucine 4.4%, glycine 5%, threonine 4.5%, serine 4.4%, tyrosine 3.2%, phenyl alanine 3.9%, methionine 2.6%, proline 3.4%, histidine 2%, cystine 1%, tryptophane 0.8%, as percentages by weight of the total weight of amino acids.

3. Method of obtaining a fish protein hydrolysate as defined in claims 1 and 2, **characterised in that** it comprises:
- pulverising of at least one protein source selected from the group comprising the fish species *Micromesistius poutassou, Clupea harengus, Scomber scombrus, Sardina pilchardus, Trisopterus esmarki, Trachurus spp., Gadus morhua, Pollachius virens, Melanogrammus aeglefinus, Coryphaenoides rupestris,* and fish belonging to the order siluriformes, in the presence of water, in such a way as to recover the flesh of said fish,
- enzymatic hydrolysis of said protein source at a temperature of between 50 and 75°C, for 1 to 5 hours, after the addition of an endopeptidase enzyme derived from *Bacillus subtilis* in such a way as to obtain a reactive mixture,
- stopping of said enzymatic hydrolysis by inactivation of said enzymes after the temperature of said reactive mixture has been raised to a level of not less than 70°C for 8 to 20 minutes,
- separation of the protein hydrolysate obtained from the remainder of the reactive mixture.

4. Method according to one of claims 3, **characterised in that** said enzymatic hydrolysis is performed with an enzyme/protein source ratio of between 0.01 and 2%, and preferably of 0.75%, and at a hydrolysis temperature of 55°C.

5. Method according to either of claims 3 and 4, **characterised in that** said pulverising of said protein source is performed on said fish in fillet form.

6. Food product **characterised in that** it comprises a fish protein hydrolysate as defined in either of claims 1 and 2.

7. Food supplement in that it comprises a fish protein hydrolysate as defined in either of claims 1 and 2.

8. Pharmaceutical composition **characterised in that** it comprises a fish protein hydrolysate as defined in claims either of claims 1 and 2.

9. Composition **characterised in that** it comprises a fish protein hydrolysate as defined in claims either of claims 1 and 2.

10. Medicament **characterised in that** it comprises a fish protein hydrolysate as defined in claims either of claims 1 and 2.

11. Use of a fish protein hydrolysate as defined in either of claims 1 and 2 for the production of a medicament intended for the treatment or prevention of a disorder selected from osteoporosis, bone demineralisation, calcium malabsorption, malabsorption of vitamin D, and bone metabolism disorders.

12. Use according to claim 11 in which the osteoporosis is post-menopausal.

13. Fish protein hydrolysate according to either of claims 1 and 2, for use thereof in the treatment or prevention of a disorder selected from osteoporosis, bone demineralisation, calcium malabsorption, malabsorption of vitamin D, and bone metabolism disorders.

14. Fish protein hydrolysate for use thereof according to claim 13, **characterised in that** the osteoporosis is post-menopausal.

15. Fish protein hydrolysate for use thereof according to claim 13, **characterised in that** the treatment or prevention of said disorder includes the stimulation of osteoblast growth and the inhibition of osteoclast growth.
